# EUROPEAN PATENT APPLICATION

(11) **EP 4 369 317 A1**
(43) Date of publication of application: **15.05.2024**
(21) Application number: 23209140.5
(22) Date of filing: 10.11.2023
(51) Int. Cl.: G07F 17/00, G07F 11/64, G06Q 10/087, G16H 20/13

(54) **SYSTEM AND METHOD FOR DISPENSING ORDERS**

(30) Priority: 11.11.2022 US 202263424735 P
(71) Applicant: RXSAFE LLC, San Marcos, California 90269 (US)
(72) Inventor: HOLMES, William K, San Diego, CA 92131 (US)
(74) Representative: Barker Brettell LLP

(57) **Abstract**

System and method for dispensing orders. One embodiment provides a pharmacy order delivery and transport system (34) including an enclosure (42) and a transport assembly (38) provided in the enclosure (42) and configured to transport a plurality of filled prescription orders. The transport assembly (38) includes a track (66) extending continuously throughout the enclosure (42) and a plurality of transport blocks (54) connected together to form a continuous loop. Each transport block (54) of the plurality of transport blocks (54) includes a transport hook (58) and a wheel (62) configured to move along the track (66). The transport hook (58) is configured to receive a storage bag (70) containing a filled prescription order of the plurality of filled prescription orders. The transport assembly (38) also includes a transport actuator (142) configured to drive the plurality of transport blocks (54) along the track (66).

## Description

### CROSS-REFERENCE TO RELATED APPLICATIONS

This application claims priority to U.S. Provisional Patent Application No. 63/424,735, filed November 11, 2022, the entire contents of which are incorporated by reference herein.

### FIELD OF THE INVENTION

The present technology relates to systems and methods for dispensing orders. More particularly, the present technology relates to systems and methods for dispensing prescription orders in a retail setting, such as a pharmacy.

### SUMMARY

Pharmacies currently use open shelves to store prepared prescription orders. When a customer arrives for picking up a prescription order, a technician manually searches for the order and provides the prescription order to the customer. The ability of the pharmacy to handle a large number of customer orders depends on the number of technicians and technician operated stations that are available to dispense orders to the customers. Additionally, many prescriptions contain controlled substances. Storing the prescription orders in open shelves may present security issues. For example, a technician may replace pills with replicas resulting in the wrong pills being provided to a customer.

Accordingly, there is a need for an efficient and secure system and method for dispensing orders.

One embodiment provides a pharmacy order delivery and transport system including an enclosure and a transport assembly provided in the enclosure and forming a continuous loop. The transport assembly is movable through the enclosure and configured to transport a plurality of filled prescription orders. The pharmacy order delivery and transport system also includes a transport actuator configured to drive the transport assembly, a dispensing opening provided on the enclosure, and an electronic processor system coupled to the transport actuator. The electronic processor is configured to receive a selection of a filled prescription order from the plurality of filled prescription orders for dispensing and determine a location of the filled prescription order on the transport assembly. The electronic processor is also configured to operate, using the transport actuator, the transport assembly to align the location of the filled prescription order with the dispensing opening, and dispense, via the dispensing opening, the filled prescription order.

Another embodiment provides a pharmacy order delivery and transport system including an enclosure and a transport assembly provided in the enclosure and configured to transport a plurality of filled prescription orders. The transport assembly includes a track extending continuously throughout the enclosure and a plurality of transport blocks connected together to form a continuous loop. Each transport block of the plurality of transport blocks includes a transport hook and a wheel configured to move along the track. The transport hook is configured to receive a storage bag containing a filled prescription order of the plurality of filled prescription orders. The transport assembly also includes a transport actuator configured to drive the plurality of transport blocks along the track.

Various embodiments, examples, aspects, and features are set forth in the description below and the accompanying drawings. Other embodiments, examples, aspects, features, objects and advantages of the techniques described in this disclosure will be apparent by consideration of the detailed description and accompanying drawings.

### BRIEF DESCRIPTION OF THE DRAWINGS

The accompanying figures, where like reference numerals refer to identical or functionally similar elements throughout the separate views, which together with the detailed description below are incorporated in and form part of the specification and serve to further illustrate various embodiments, examples, aspects, and features that include the claimed subject matter, and to explain various principles and advantages of aspects of those embodiments, examples, aspects, and features.
FIG. 1 is a perspective view of a pharmacy including an order delivery and storage system in accordance with some examples.
FIG. 2 is another perspective view of the pharmacy of FIG. 1 in accordance with some examples.
FIG. 3 is an enlarged perspective view of a portion of the pharmacy of FIG. 1 in accordance with some examples.
FIG. 4 is an enlarged perspective view of a portion of the pharmacy of FIG. 1 in accordance with some examples.
FIG. 5 is an enlarged perspective view of a portion of the pharmacy of FIG. 1 in accordance with some examples.
FIG. 6 is an enlarged perspective view of a portion of the pharmacy of FIG. 1 illustrating a stocking station in accordance with some examples.
FIG. 7 is an enlarged perspective view of the stocking station of the order delivery and storage system of FIG. 1 in accordance with some examples.
FIG. 8 is an enlarged perspective view of a portion of a transport assembly of the order delivery and storage system of FIG. 1 in accordance with some examples.
FIG. 9 is an enlarged perspective view of a portion of the transport assembly of the order delivery and storage system of FIG. 1 in accordance with some examples.
FIG. 10 is an enlarged perspective view of a portion of the transport assembly of the order delivery and storage system of FIG. 1 in accordance with some examples.
FIG. 11 is a simplified block diagram of the order delivery and storage system of FIG. 1 in accordance with some examples.
FIG. 12 is a flowchart of a method for operating the transport assembly of the order delivery and storage system of FIG. 1 in accordance with some examples.
FIG. 13 is a flowchart of a method for stocking the order delivery and storage system of

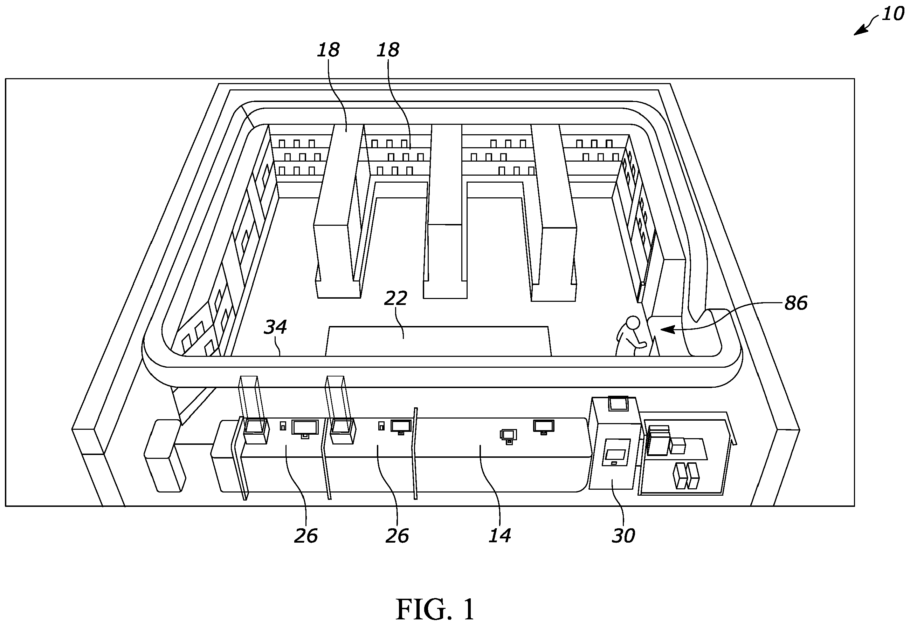

FIG. 1 in accordance with some examples.

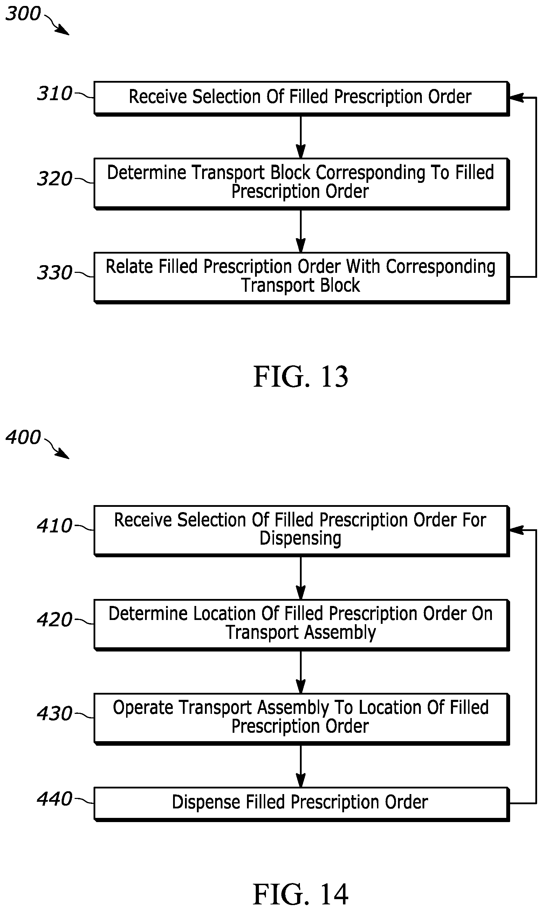

FIG. 14 is a flowchart of a method for dispensing order using the order delivery and storage system of FIG. 1 in accordance with some examples.

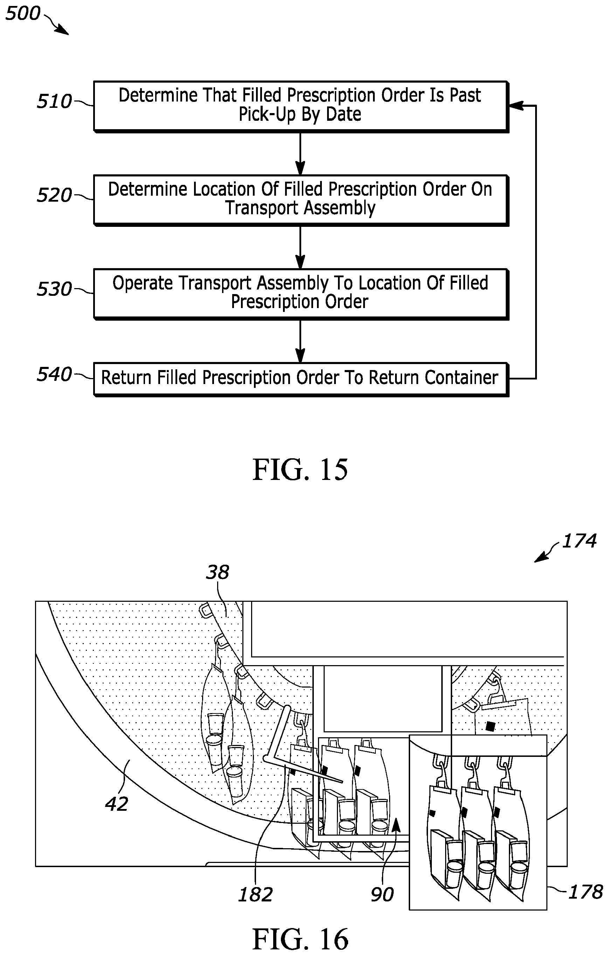

FIG. 15 is a flowchart of a method for returning orders using the order delivery and storage system of FIG. 1 in accordance with some examples.
FIG. 16 is an enlarged perspective view of the stocking station of the order delivery and storage system of FIG. 1 in accordance with some examples.

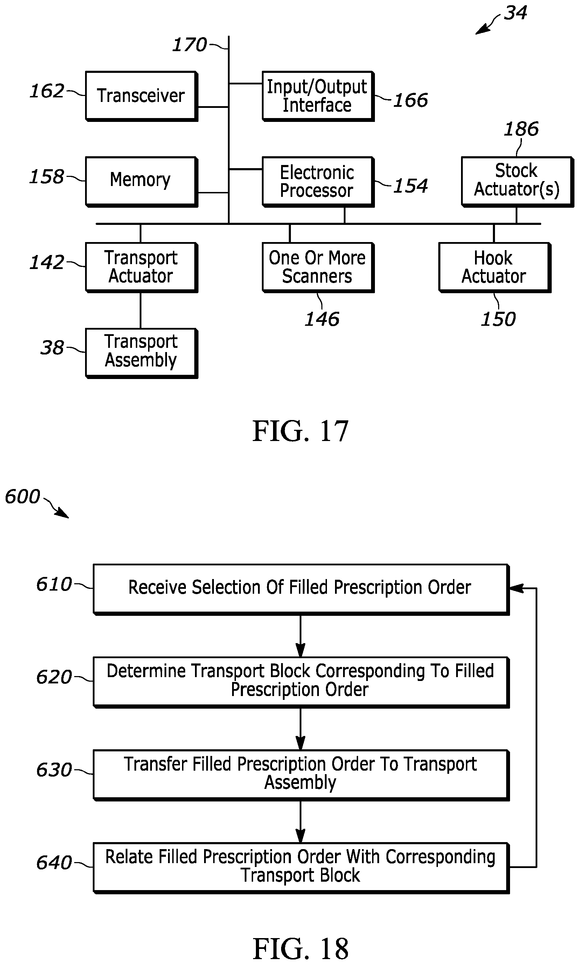

FIG. 17 is a simplified block diagram of the order delivery and storage system of FIG. 1 in accordance with some examples.
FIG. 18 is a flowchart of a method for stocking the order delivery and storage system of FIG. 1 in accordance with some examples.

Skilled artisans will appreciate that elements in the figures are illustrated for simplicity and clarity and have not necessarily been drawn to scale. For example, the dimensions of some of the elements in the figures may be exaggerated relative to other elements to help to improve understanding of examples of the present technology.

The apparatus and method components have been represented where appropriate by conventional symbols in the drawings, showing only those specific details that are pertinent to understanding the embodiments, examples, aspects, and features so as not to obscure the disclosure with details that will be readily apparent to those of ordinary skill in the art having the benefit of the description herein.

### DETAILED DESCRIPTION

Before any embodiments, examples, aspects, and features are explained in detail, it is to be understood that those embodiments, examples, aspects, and features are not limited in their application to the details of construction and the arrangement of components set forth in the following description or illustrated in the following drawings. Other embodiments, examples, aspects, and features are possible and are capable of being practiced or carried out in various ways.

Also, it is to be understood that the phraseology and terminology used herein is for the purpose of description and should not be regarded as limiting. The terms "mounted," "connected," and "coupled" are used broadly and encompass both direct and indirect mounting, connecting, and coupling. The terms "connected" and "coupled" are not restricted to physical or mechanical connections or couplings, and can include electrical connections or couplings, whether direct or indirect. Electronic communications and notification described herein may be performed using any known or future-developed means including wired connections, wireless connections, etc.

For ease of description, some or all of the example systems presented herein are illustrated with a single exemplar of each of its component parts. Some examples may not describe or illustrate all components of the systems. Other embodiments, examples, aspects, and features may include more or fewer of each of the illustrated components, may combine some components, or may include additional or alternative components.

FIGS. 1-10 illustrate a retail location, or store, including a pharmacy 10. The pharmacy 10 includes a counter 14, walls, and other structure that separate pharmacists and technicians working in the pharmacy 10 from customers in the retail location. In the example illustrated, the pharmacy 10 includes a plurality of storage racks 18 for storing pharmaceuticals, filled prescription orders, and other controlled substances. In some examples, one or more of the storage racks 18 may be replaced with a pharmaceutical storage and retrieval device as described in U.S. Patent No. 8,849,445, the entire contents of which are incorporated by reference herein.

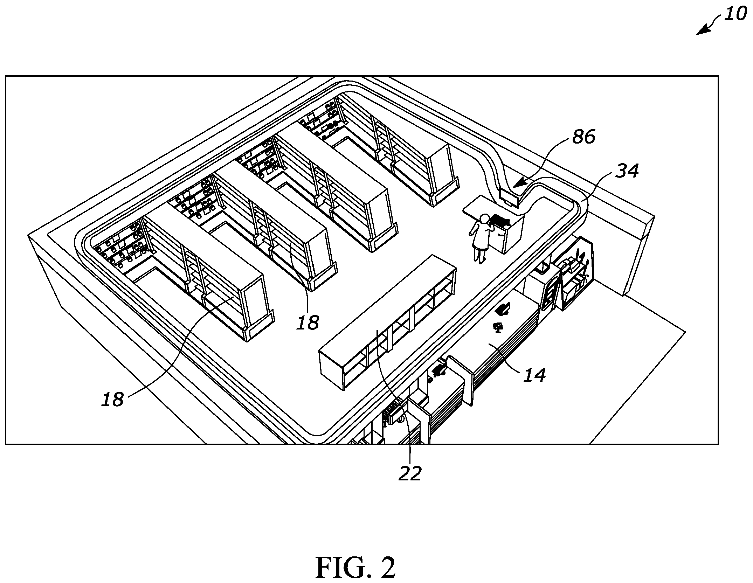

As shown in FIGS. 1 and 2, the pharmacy 10 includes a filling station 22 for preparing and filling prescription orders. When a prescription is received by the pharmacy, a pharmacist or technician gathers bulk containers including the pharmaceuticals listed on the prescription from the storage racks 18. The pharmacist or technician then uses the filling station 22 to prepare the prescription order based on the prescription. In some examples, the pharmacy 10 includes an automatic packager as described in U.S. Patent No. 10,583,941, the entire contents of which are incorporated by reference herein. Instead of packaging prescription orders by hand, the prescription orders may be packaged using the automatic packager.

As shown in FIGS. 1-3, the counter 14 includes a plurality of dispensing stations 26. The dispensing stations 26, or point of sale areas, are used by pharmacists or technicians to receive the prescription from a customer and process the prescription. Once the prescription is prepared by the pharmacist or the technician, the filled prescription order is retrieved and sold to the customer at the dispensing stations 26. In some examples, the pharmacy 10 also includes a self-dispensing kiosk 30 where a customer may pick up an order. The self-dispensing kiosk 30 may dispense a filled prescription order based on prescription details entered by the customer.

When a prescription order is prepared, the filled prescription order is stored in one of the storage racks 18 for later retrieval. The filled prescription order may be stored according to customer name, order identification number, or the like. When a customer arrives to pick up the order, the pharmacist or technician manually searches for the order using the name or number and retrieves the order for sale. However, this process is inefficient and error prone. Additionally, the filled prescription orders are stored in open storage racks 18. This presents a security issue as the filled prescription order may be tampered with prior to sale.

An order delivery and storage system 34 may be used in the pharmacy 10 to provide a secure and efficient system to store and deliver filled prescription orders. In the example illustrated, the order delivery and storage system 34 may be provided above the storage racks 18 and the counter 14 and fixed to the walls and/or ceiling of the pharmacy 10. For example, the enclosure of the order delivery and storage system 34 may be fixed to a ceiling or walls of a building including the pharmacy 10. The order delivery and storage system 34 may be offset from the ceiling to reduce interference with overhead lights, sprinkler systems, and the like. For example, the order delivery and storage system 34 may be offset about one foot from the ceiling. In other embodiments, the order delivery and storage system 34 may be offset a different amount from the ceiling or may be mounted directly on the ceiling. In some examples, the order delivery and storage system 34 may be provided at a different location and/or in a different configuration (for example, along a side wall, along the floor, or the like).

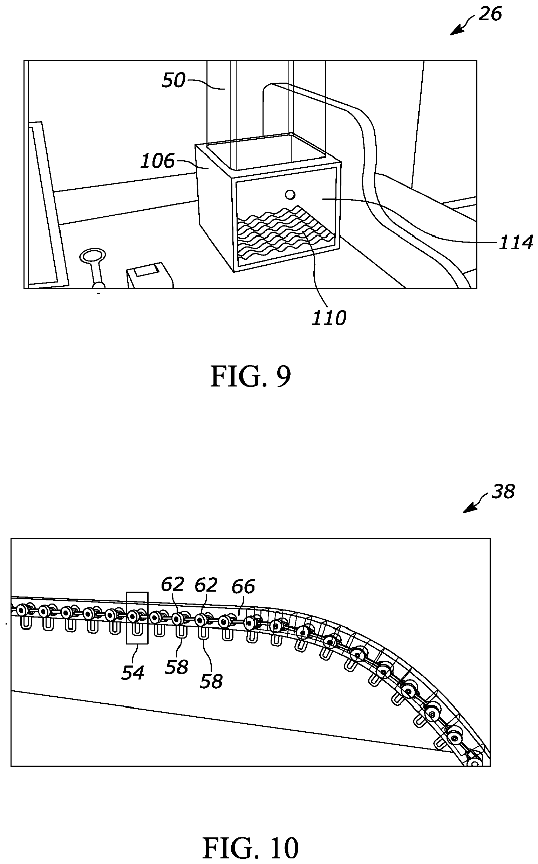

The order delivery and storage system 34 includes a transport assembly 38 (see FIG. 10) provided in an enclosure 42. The enclosure 42 may be made of modular sections having different configurations (e.g., straight sections, bent sections, etc.) that can be assembled in any desired configuration to fit within a given pharmacy. In some examples, the enclosure 42 is made of opaque material such that the contents of the order delivery and storage system 34 may not be seen through the enclosure 42. In other examples, the enclosure 42 is made of clear or translucent material such that the contents of the order delivery and storage system 34 can be seen from outside the enclosure 42. The clear or translucent material also allows light to pass through, reducing the amount of light being blocked by the order delivery and storage system 34. The enclosure 42 may be provided with doors 44 that can be opened to access the contents of the order delivery and storage system 34. The doors 44 may be provided every few feet on the enclosure (e.g., plurality of spaced apart doors 44) such that the order delivery and storage system 34 may be serviced in case of an error or stoppage. In some embodiments, the doors 44 may be locked and require a key, code, fob, or the like to be opened.

In the example illustrated, the order delivery and storage system 34 includes a plurality of dispensing openings 46 that dispense filled prescription orders to the dispensing stations 26 and the self-dispensing kiosk 30. The dispensing openings 46 are provided above the dispensing stations 26 and the self-dispensing kiosk 30. A plurality of dispensing tubes 50 connect the dispensing openings 46 to the dispensing stations 26 and the self-dispensing kiosk 30. In some embodiments, only a single dispensing opening 46 and/or a single dispensing tube 50 may be provided that is shared between the dispensing stations 26 and the self-dispensing kiosk 40. In some embodiments, only two dispensing openings 46 and dispensing tubes 50 are provided such that one dispensing opening 46 and dispensing tube 50 are provided to the self-dispensing kiosk 30, while the dispensing stations 26 share the other dispensing opening 46 and dispensing tube 50. Other configurations including more or fewer dispensing openings 46 and dispensing tubes 50 may also be used without deviating from the scope of the disclosure.

Referring to FIG. 10, the illustrated transport assembly 38 includes a plurality of modular transport blocks 54 that are connected together to form the transport assembly 38. Each transport block 54 includes a transport hook 58 and one or more wheels 62. Each transport block 54 is connected to the next transport block 54 such that the connected transport blocks 54 can be moved together along different axes. For example, the connection between the transport block 54 allows for tolerances when the transport blocks 54 are moved around turns and dips in the order delivery and storage system 34. The one or more wheels 62 move along a track 66 of the transport assembly 38. The number of transport blocks 54 used may depend on the size and capacity requirements of the pharmacy 10. Transport blocks 54 may be added and removed from the order delivery and storage system 34 as desired and based on changing requirements of the pharmacy 10. The transport assembly 38 may be driven around the track 66 using an actuator (for example, a motor, an engine, or the like). In some examples, the transport blocks 54 are connected to form a continuous loop or endless transport assembly 38 that can be driven from a single location. In other examples, the transport assembly 38 may include transport blocks that are fixed to a chain. In such embodiments, the chain may be driven throughout the enclosure 42 by the actuator.

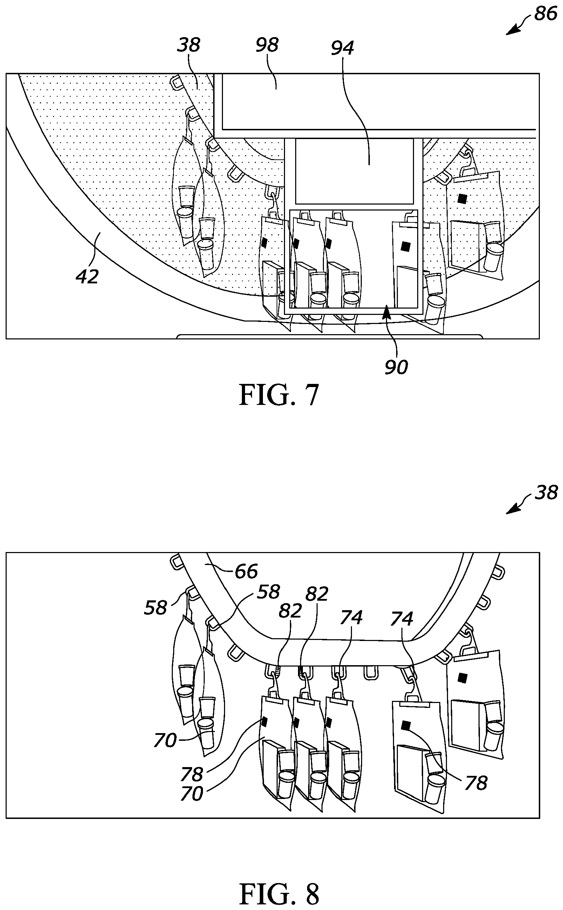

Referring to FIGS. 7-8, the transport hooks 58 are configured to receive storage bags 70. Each storage bags 70 includes a bag hook 74 that is used to place the storage bag 70 on one of the transport hooks 58. Each storage bag 70 may store one prescription order. Alternatively, each storage bag 70 may store multiple prescription orders, such as multiple prescription orders for the same customer. A bag bar code 78 or other indicia (e.g., QR code, RF tag, etc.) to uniquely identify the storage bag 70 is provided on each storage bag 70. A hook bar code 82 or other indicia (e.g., QR code, RF tag, etc.) to uniquely identify each transport hook 58 is also provided on each transport hook 58 of the transport assembly 38. The transport hooks 58 may be identified sequentially. For example, each subsequent transport hook 58 may have a sequential identifier such that the order delivery and storage system 34 can identify the location of each subsequent transport hook 58 when a location of one transport hook 58 is known. In some examples, the location of storage bags 70 may be tracked using only the indicia on the storage bags 70 or independent of tracking the location of the transport assembly 38. The order delivery and storage system 34 may store the location of each storage bag 70 in relation to other storage bags 70. In these examples, the hook bar code 82 or other indicia on the transport hook 58 may not be needed or may not be used.

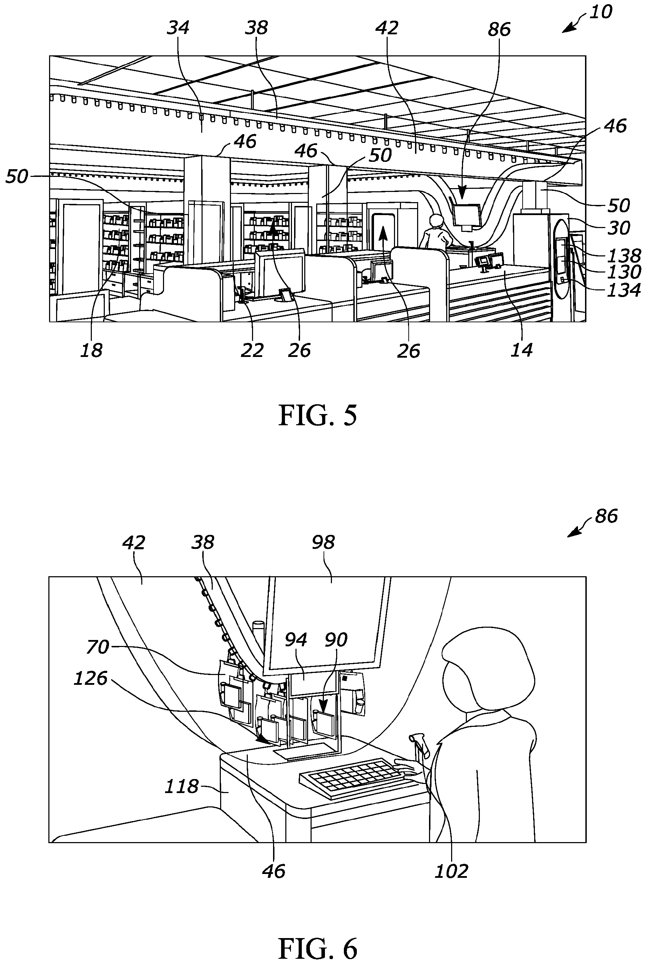

Referring to FIGS. 6-8, the order delivery and storage system 34 includes a stocking station 86. The transport assembly 38 includes a dip at the location of the stocking station 86 such that the transport assembly 38 can be accessed by a user at a height level corresponding to the user. The stocking station 86 includes a stocking opening 90 covered by a stocking door 94 provided on the enclosure 42. In some examples, the stocking door 94 can be manually moved to open and close the stocking opening 90. In some examples, the stocking door 94 is automatically operated using a door actuator to open and close the stocking opening 90. In some examples, the stocking opening 90 is sized such that only a single transport hook 58 or a single storage bag 70 is accessible to a pharmacist or technician. A stocking workstation 98 is provided at the stocking station 86. The stocking workstation 98 includes a stock scanner 102, for example, a bar code scanner that can be used to scan the bar codes or other identifying information on the transport hooks 58, the storage bags 70, the prescriptions, and/or the like. In some examples, the stocking station 86 also includes a return container 118 to receive filled prescription orders that have not been picked up. The return container 118 includes a return opening 126 aligned with a dispensing opening 46 of the enclosure 42. In some examples, a return door is provided over the return opening 126 that is automatically operated by the order delivery and storage system 34 when a storage bag 70 is being returned to the return container 118. In other example, the return container 118 may be provided at a different location in the pharmacy 10 separate from the stocking station 86.

Referring to FIG. 9, the dispensing station 26 includes a dispensing chamber 106 to receive a filled prescription order from the order delivery and storage system 34. A dispensing tube 50 connects a corresponding dispensing opening 46 in the enclosure 42 to the dispensing chamber 106. The dispensing chamber 106 includes a padded base 110 to pad the filled prescription order when the filled prescription order is received from the dispensing opening 46. A door 114 may be provided on the dispensing chamber 106 to access the contents of the dispensing chamber 106.

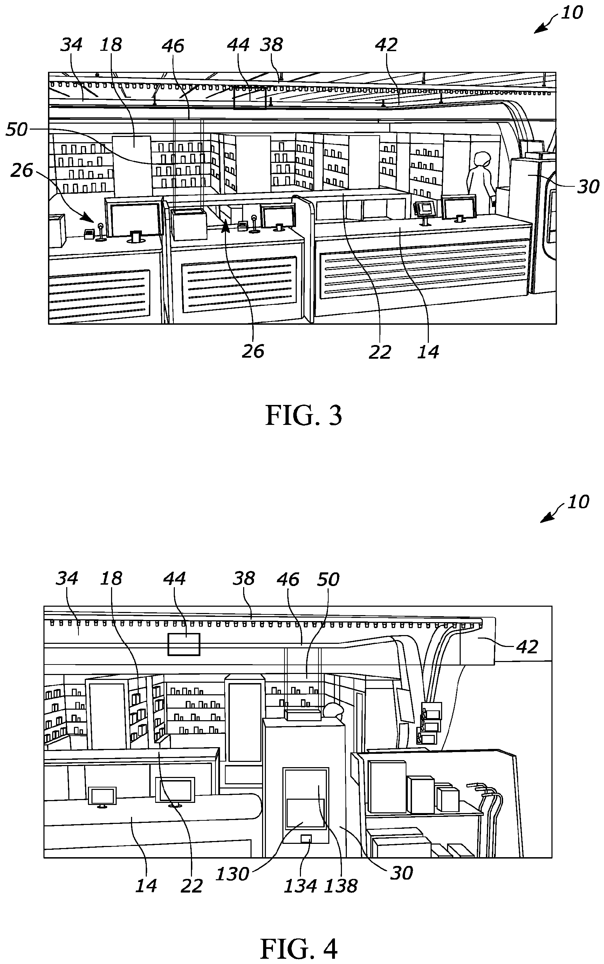

Referring to FIG. 4, the kiosk 30 includes a display screen 130 and an input mechanism 134. The input mechanism 134 may be used by a customer to scan a prescription or to provide identification information related to the customer or the order being picked up by the customer. The display screen 130 is used to present information to the customer related to, for example, the filled prescription order, payment information, and/or the like. In the example illustrated, a kiosk dispensing chamber 138 is provided on the kiosk 30 above the display screen 130. In other examples, the kiosk dispensing chamber 138 may be provided at other locations on the kiosk 30. A dispensing tube 50 connects a corresponding dispensing opening 46 in the enclosure 42 to the kiosk dispensing chamber 138. The kiosk dispensing chamber 138 may be implemented similar to the dispensing chamber 106, for example, including an opening, a door, and a padded base to receive the filled prescription order.

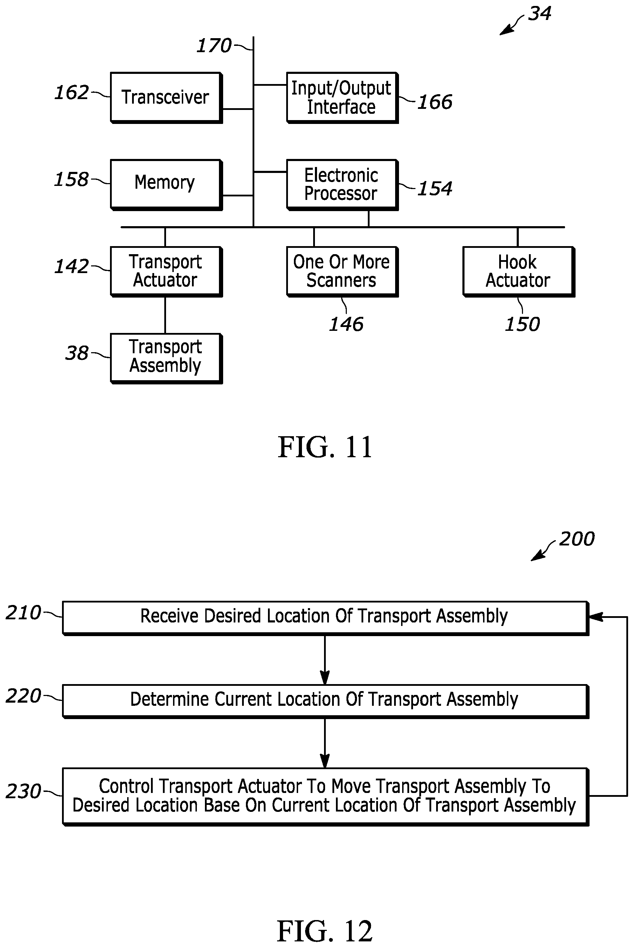

FIG. 11 is a simplified block diagram of one example of the order delivery and storage system 34. In the example illustrated, the order delivery and storage system 34 includes the transport assembly 38, a transport actuator 142, one or more scanners 146, a plurality of hook actuators 150, an electronic processor 154, a memory 158, a transceiver 162, and an input/output interface 166. The transport actuator 142 includes, for example, a motor, an engine, or the like that drives the transport assembly 38. The transport actuator 142 may be provided in the enclosure 42 at a location within the pharmacy 10 than can be accessed for servicing. The electronic processor 154 controls the transport actuator 142 to drive the transport assembly 38. The transport actuator 142 may impart bi-directional movement to the transport assembly 38, for example, to rotate the transport assembly 38 in both a clockwise direction and an anti-clockwise direction.

The one or more scanners 146 include, for example, bar code scanners or the like that can be used to identify the location of the transport assembly 38. The location of the transport assembly 38 corresponds to the identity of the transport block 54 that is in front of the scanner 146. In one example, one scanner 146 is provided at a location within the enclosure 42. The scanner 146 identifies the transport block 54 using, for example, a bar code provided on the transport hook 58 of the transport block 54. The location of every other transport block 54 may be determined based on the transport block 54 in front of the scanner 146 based on a pre-stored mapping of the transport blocks 54 stored in the memory 158. In some examples, a plurality of scanners 146 are provided at multiple locations in the enclosure 42. For example, a scanner 146 is provided at each of the stocking opening 90 and the plurality of dispensing openings 46 to identify the transport block 54 in front of the scanners 146. As noted above, in some examples, the location of the storage bags 70 may be tracked independent of the transport assembly 38. In these examples, each of the plurality of scanners 146 may continuously scan the storage bags 70 as they pass by the plurality of scanners 146 to ensure that no storage bags 70 have been knocked off a transport hook 58 between a previous scanner 146 and the currents scanner 146. The electronic processor 154 may generate an alert when a discrepancy in the storage bags 70 (for example, a missing storage bag 70) is determined using the plurality of scanners 146 as noted above. The electronic processor 154 receives the scanned information from the one or more scanners 146 and controls the transport actuator 142 to drive the transport assembly 38 accordingly. In some examples, the one or more scanners 146 also includes the stock scanner 102 and/or additional bar code scanners provided at the dispensing stations 26 and the kiosk 30.

The plurality of hook actuators 150 are provided at each of the stocking opening 90 and the plurality of dispensing openings 46. The hook actuators 150 include, for example, a solenoid, a motor, or the like that are configured to knock the storage bags 70 off the transport hooks 58. In one example, the hook actuator 150 includes a motor or solenoid to separate the transport hook 58 such that the storage bag 70 falls off the transport hook 58. In another example, the hook actuator 150 drives a bar that pushes the bag hook 74 such that the storage bag 70 is knocked off the transport hook 58. The plurality of the hook actuators 150 are controlled by the electronic processor 154 to dispense or return the storage bags 70 from the transport assembly 38.

In the example illustrated, the electronic processor 154, the memory 158, the transceiver 162, and the input/output interface 166 communicate over one or more control and/or data buses (for example, a communication bus 170). The electronic processor 154 is connected to the transport actuator 142 and the plurality of hook actuators 150 to control the transport actuator 142 and the plurality of hook actuators 150. The electronic processor 154 is also connected to the one or more scanners 146 to receive identification information from the one or more scanners 146. In some examples, the electronic processor 154 is implemented as a microprocessor with separate memory, such as the memory 158. In other examples, the electronic processor 154 may be implemented as a microcontroller (with memory 158 on the same chip). In other examples, the electronic processor 154 may be implemented using multiple processors (in some cases located remote from one another). In addition, the electronic processor 154 may be implemented partially or entirely as, for example, a field-programmable gate array (FPGA), an applications specific integrated circuit (ASIC), an x86 processor, and the like and the memory 158 may not be needed or be modified accordingly. In the example, illustrated, the memory 158 includes non-transitory, computer readable memory that stores instructions that are received and executed by the electronic processor 154 to carry out the functionality of the order delivery and storage system 34 described herein. The memory 158 may include, for example, a program storage area and a data storage area. The program storage area and the data storage area may include combinations of different types of memory, such as read-only memory and random-access memory. In some examples, the order delivery and storage system 34 includes one electronic processor 154 and/or a plurality of electronic processors 154 in a computer cluster arrangement, one or more of which may be executing none, all, or a portion of the applications of the order delivery and storage system 34. The one or more electronic processors 154 may be collocated in a single device or may be distributed between different devices that may be collocated or separated. An electronic processor system is used herein to refer to the one or more electronic processors. Each of the functions described herein is performed by the electronic processor system. A function performed by the electronic processor system may all be performed by a single electronic processor or may be performed by (i.e., distributed between) multiple electronic processors.

The transceiver 162 enables wired or wireless communication between the electronic processor 154 and other systems. In one example, the transceiver 162 enables wired or wireless communication between the electronic processor 154 and the stocking workstation 98, the kiosk 30, and/or the workstations at the dispensing stations 26. In other examples, one or more of the stocking workstation 98, the kiosk 30, and the workstations at the dispensing stations 26 are implemented as part of the same system including the electronic processor 154. The input/output interface 166 includes an input mechanism (for example, a keyboard, a mouse, and the like), an output mechanism (for example, a display, a speaker, and the like), or a combined input/output mechanism (for example, a touch screen display). In some examples, the input/output interface may be implemented as one or more of the stocking workstation 98, the kiosk 30, and the workstations at the dispensing stations 26.

FIG. 12 illustrates a flowchart of an example method 200 for operating the transport assembly 38 of the order delivery and storage system 34. In the example illustrated, the method 200 includes receiving, using the electronic processor 154, a desired location of the transport assembly 38 (at block 210). In one example, the transport assembly 38 may include 2000 transport blocks 54 each sequentially assigned a number from 1 to 2000. In this example, the location of the transport assembly 38 corresponds to the number assigned to the transport block 54 that is currently in front of the one or more scanners 146. For example, if the transport block 54 assigned with the number 1000 is in front of the one or more scanners 146, then the current location of the transport block 54 may be determined as 1000. A desired location of the transport assembly 38 corresponds to the placing the transport block 54 having the desired assigned number in front of the one or more scanners 146. In other examples, rotary encoders or other sensors can be used to determine or track the location of the transport assembly 58. The desired location of the transport assembly 38 may be specified by any of the following methods described below.

The method 200 includes determining, using the electronic processor 154, a current location of the transport assembly 38 (at block 220). As discussed above, the current location of the transport assembly 38 may correspond to the identity (for example, a number assigned) of the transport block 54 currently in front of the one or more scanners 146. The electronic processor 154 uses the one or more scanners 146 to scan a bar code of the transport block 54 currently in front of (that is, within a scanning range of) the one or more scanners 146. The electronic processor 154 determines the current location based on the scanned information received from the one or more scanners 146.

The method 200 includes controlling, using the electronic processor 154, the transport actuator 142 to move the transport assembly 38 to the desired location based on the current location of the transport assembly 38 (at block 230). As discussed above, the transport block 54 may be assigned numbers in a sequential fashion such that each subsequent transport block 54 may have a higher number than the immediately preceding transport block 54 (except for transport blocks 54 assigned numbers 1 and 2000). Accordingly, when the current location of the transport assembly 38 is determined, the location of the desired transport block 54 is also known. For example, the memory 158 may store a mapping of the transport blocks 54 such that the electronic processor 154 may determine the location of the desired transport block 54 based on the current location of the transport assembly 38 using the stored mapping. In some examples as previously discussed, the location of storage bags 70 may be stored in a mapping in relation to the location of other storage bags 70. In these examples, the one or more scanners 146 read the bag bar codes 78 to track the location of the storage bags 70 and correspondingly the transport assembly 38. The electronic processor 154 then controls the transport actuator 142 to move the transport assembly 38 to the desired location. For example, the electronic processor 154 controls the transport actuator 142 (for example, a motor) to complete a number of revolutions sufficient to move the transport assembly 38 to the desired location. The transport assembly 38 may be moved in either direction (for example, clockwise or anti-clockwise) to take the shortest path to the desired location. In some examples, the electronic processor 154 may further verify that the desired location of the transport assembly 38 is achieved using the one or more scanners 146. That is, the electronic processor 154 may determine whether the desired transport block 54 is currently in front of (that is, within a scanning range of) the one or more scanners 146. The method 200 may repeat every time a new location of the transport assembly 38 is desired.

FIG. 13 illustrates a flowchart of an example method 300 for stocking the order delivery and storage system 34. A pharmacist or technician may stock the order delivery and storage system using the stocking station 86. The method 300 includes receiving, using the electronic processor 154, a selection of a filled prescription order (at block 310). As discussed above, a pharmacist or technician fills a prescription order based on a prescription received from a customer. In some examples, the prescription order may be filled or packaged by an automatic packager. A label is attached to the filled prescription order to identify the filled prescription order. The label may include a bar code or other indicia that can be read by the electronic processor 154 using the one or more scanners 146 (for example, the stock scanner 102). In some examples, the filled prescription order with or without a label including identifying marks is placed in a storage bag 70. The storage bag 70 may include a bar code or other indicia that can be read by the electronic processor 154 using the one or more scanners 146 (for example, the stock scanner 102). During a session when the filled prescription order is selected, the storage bag 70 in which the filled prescription order is placed is scanned by the pharmacist or technician. The electronic processor 154 receives the identification information of the storage bag 70 and associates the storage bag 70 with the filled prescription order. Associating the storage bag 70 with the filled prescription order may include storing the identification information of the storage bag 70 corresponding to the identification information of the filled prescription order in the memory 158. Receiving the selection of the filled prescription order may include receiving the identification information of the filled prescription order or the identification information of the storage bag 70 including the filled prescription order. In one example, receiving the selection of the filled prescription order includes scanning, using the stock scanner 102, a barcode on the filled prescription order or the storage bag 70 including the filled prescription order.

The method 300 also includes determining, using the electronic processor 154, a transport block 54 corresponding to the filled prescription order (at block 320). In some examples, the electronic processor 154 automatically finds an empty transport block 54 to stock the filled prescription order. In other examples, the pharmacist or technician may operate the transport assembly 38 until an empty transport block 54 is at the stocking station 86 and uses the empty transport block 54 to stock the filled prescription order. To stock the filled prescription order, the empty transport block 54 may be scanned during the session when the filled prescription order is selected. The empty transport block 54 may be scanned by the pharmacist using the stock scanner 102 or automatically by the electronic processor 154 using the one or more scanners 146 provided at the stocking station 86.

The method 300 includes relating, using the electronic processor 154, the filled prescription order with the corresponding transport block 54 (at block 330). The electronic processor 154 may store a mapping in the memory 158 corresponding identification information of the filled prescription orders (or the storage bags 70 including the filled prescription orders) with the identification information of the transport blocks 54 where the filled prescription orders are stored. This mapping is available for later reference when dispensing the filled prescription order or when returning the filled prescription order. The method 300 may be repeated for every filled prescription order being stocked in the order delivery and storage system 34.

FIG. 14 illustrates a flowchart of an example method 400 for dispensing a filled prescription order using the order delivery and storage system 34. In the example illustrated, the method 400 includes receiving, using the electronic processor 154, a selection of a filled prescription order for dispensing (at block 410). The dispensing process may begin when a customer presents a prescription to be picked up. The pharmacist or technician may search using the identification information on the prescription or the identification information of the customer or patient (for example, last name) to find the filled prescription order corresponding to the prescription. The pharmacist or technician may enter the identification information into a dedicated keypad or input device that communicates with the order delivery and storage system 34. Alternatively, the order delivery and storage system 34 may be integrated with the pharmacy's existing computer system such that the pharmacist or technician may enter the identification information into a corresponding pharmacy computer. The selection of the filled prescription order may be received at a workstation at the dispensing station 26. In some embodiments, the customer may provide identification information at the kiosk 30. The kiosk 30 may display the corresponding filled prescription order information on the display screen 130 of the kiosk for selection by the customer. In some embodiments, the one or more scanners 146 (for example, a barcode scanner at the dispensing station 26 or the kiosk 30) may be used to scan the prescription information to select the corresponding filled prescription order for dispensing.

The method 400 includes determining, using the electronic processor 154, a location of the filled prescription order on the transport assembly 38 (at block 420). As discussed above, when the filled prescription order is stocked in the order delivery and storage system 34, the electronic processor 154 stores a mapping of the location of the stocked filled prescription orders. The electronic processor 154 may refer to the mapping to determine the location of the filled prescription order on the transport assembly 38. The location of the filled prescription order is, for example, identification information of the transport block 54 on which the filled prescription order is stored.

The method 400 includes operating, using the electronic processor 154, the transport assembly 38 of the order delivery and storage system 34 to the location of the filled prescription order on the transport assembly 38 (at block 430). Operating the transport assembly 38 to the location of the filled prescription order includes, for example, aligning the transport block 54 having the filled prescription order with the dispensing opening 46 and/or the hook actuator 150 of the dispensing opening 46 corresponding to the dispensing station 26 or the kiosk 30 at which the selection of the filled prescription order for dispensing is received. The electronic processor 154 may execute method 200 to operate the transport assembly 38 to the location of the filled prescription order, where the desired location of method 200 corresponds to the location of the filled prescription order.

The method 400 includes dispensing, using the electronic processor 154, the filled prescription order (at block 440). The filled prescription order is dispensed when the transport assembly is operated to or at the location of the filled prescription order. The electronic processor 154 controls the hook actuator 150 to knock the filled prescription order off the transport block 54 or otherwise remove (e.g., lift, slide, pull, etc.) the filled prescription order from the transport block 54. The filled prescription order is provided to the customer after being picked up from the dispensing chamber 106 of the dispensing station 26 or may be picked up directly by the customer at the kiosk dispensing chamber 138. The electronic processor 154 may then mark the corresponding transport block 54 as empty and ready to be used to store the next filled prescription order at the stocking station 86. The method 400 may be repeated for every filled prescription order that is to be dispensed.

Many jurisdictions have requirements to return or dispose filled prescription orders that have not been picked up in, for example, 14 days. Additionally, filled prescription orders that are not picked up may take up space in the order delivery and storage system 34, increasing costs for the pharmacy. Therefore, it may be desirable to return filled prescription orders when the filled prescription orders are not picked within a pick-up time window. FIG. 15 illustrates a flowchart of an example method 500 for returning a filled prescription order using the order delivery and storage system 34. In the example illustrated, the method 500 includes determining, using the electronic processor 154, that a filled prescription order is past a pick-up by date (at block 510). When the filled prescription orders are stocked in the order delivery and storage system 34, a pick-up by date may be assigned to the filled prescription order. The pick-up by date may be set as 14 days from the date the filled prescription order was prepared or stocked. In other embodiments, the pick-up by date may be set as a shorter or longer timeframe (e.g., 7 days, 21 days, 30 days, etc.). In some embodiments, the electronic processor 154 may store the pick-up by date corresponding to the identification information of the filled prescription order and/or the location of the filled prescription order in a mapping (for example, a relational database) stored in the memory 158.

The method 500 includes determining, using the electronic processor 154, a location of the filled prescription order on the transport assembly 38 (at block 520). As discussed above, when the filled prescription order is stocked in the order delivery and storage system 34, the electronic processor 154 stores a mapping of the location of the stocked filled prescription orders. The electronic processor 154 may refer to the mapping to determine the location of the filled prescription order on the transport assembly 38. The location of the filled prescription order is, for example, identification information of the transport block 54 on which the filled prescription order is stored.

The method 500 includes operating, using the electronic processor 154, the transport assembly 38 of the order delivery and storage system 34 to the location of the filled prescription order on the transport assembly 38 (at block 530). Operating the transport assembly 38 to the location of the filled prescription order includes, for example, aligning the transport block 54 having the filled prescription order with the return opening 126 and/or the hook actuator 150 corresponding to the return container 118. The electronic processor 154 may execute method 200 to operate the transport assembly 38 to the location of the filled prescription order, where the desired location of method 200 corresponds to the location of the filled prescription order.

The method 500 includes returning, using the electronic processor 154, the filled prescription order to the return container 118 (at block 540). The electronic processor 154 controls the hook actuator 150 to knock, or otherwise remove, the filled prescription order off the transport block 54 and into the return container 118. In some embodiments, a camera or other sensor may be provided in the return container 118 or at the return opening 126 to verify that the filled prescription order was returned to the return container 118. The pharmacist or technician may then return any unexpired and/or usable pharmaceuticals from the returned filled prescription order back to bulk containers on the storage racks 18 and may dispose any expired or unusable pharmaceuticals. The method 500 may be repeated for every filled prescription order that is past its pick-up by date.

FIG. 16 illustrates another example implementation of a stocking station 174. The stocking station 174 includes similar features as the stocking station 86 and is additionally configured to stock the order delivery and transport assembly 34 without stopping the order and transport assembly 38. The stocking station 174 includes a stock holder 178 that temporarily holds storage bags 70 before they are loaded into the order delivery and transport assembly 34. The stock holder 178 may also be referred to as a staging area. The stock holder may include hooks similar to the hooks 58 to temporarily hold the storage bags 70. The stocking station 174 also includes a stocking mechanism 182 that automatically moves the storage bags 70 from the stock holder 178 to the order delivery and transport assembly 34. The stocking mechanism 182 may be implemented as, for example, a robotic arm or robotic gantry assembly.

In another example, the transport assembly 38 does not include a dip such that the transport assembly 38 forms a continuous loop along the same height at or near the ceiling. In this example, a separate stock transport loop may be provided to facilitate stocking of the storage bags 70 into the order and transport assembly. The stock transport loop may be formed at the location of the stocking station 86, 174 and includes a stocking station at the user height level similar to the stocking station 86. The stock transport loop includes a stock transport assembly similar to the transport assembly 38. The stock transport loop may be stocked similarly as described with the stocking station 86. The stock transport assembly then moves the stocked hooks upwards closer to the transport assembly 38. A stocking mechanism 182 may then be utilized to move the storage bags 70 from the stock transport loop to the transport assembly 38.

FIG. 17 is a simplified block diagram of one example of the order delivery and storage system 34. As illustrated in FIG. 17, in addition to all of the components as shown in FIG. 11, the order delivery and storage system 34 also includes a stock actuator(s) 186. The stock actuator(s) 186 may include actuator(s) to drive the stocking mechanism 182 and/or the stock transport assembly. The stock actuator(s) 186 includes, for example, a motor, an engine, or the like that drives the stocking mechanism 182 and/or the stock transport assembly. The stock actuator(s) 186 may be provided in the enclosure 42 at a location within the pharmacy 10 that can be accessed for servicing. The electronic processor 154 controls the stock actuator(s) 186 to drive the stocking mechanism 182 and/or the stock transport assembly.

FIG. 18 illustrates a flowchart of an example method 600 for stocking the order delivery and storage system 34. A pharmacist or technician may stock the order delivery and storage system using the stocking station 174. The method 300 includes receiving, using the electronic processor 154, a selection of a filled prescription order (at block 610). As discussed above, a pharmacist or technician fills a prescription order based on a prescription received from a customer. In some examples, the prescription order may be filled or packaged by an automatic packager. A label is attached to the filled prescription order to identify the filled prescription order. The label may include a bar code or other indicia that can be read by the electronic processor 154 using the one or more scanners 146 (for example, the stock scanner 102). In some examples, the filled prescription order with or without a label including identifying marks is placed in a storage bag 70. The storage bag 70 may include a bar code or other indicia that can be read by the electronic processor 154 using the one or more scanners 146 (for example, the stock scanner 102). During a session when the filled prescription order is selected, the storage bag 70 in which the filled prescription order is placed is scanned by the pharmacist or technician. The electronic processor 154 receives the identification information of the storage bag 70 and associates the storage bag 70 with the filled prescription order. Associating the storage bag 70 with the filled prescription order may include storing the identification information of the storage bag 70 corresponding to the identification information of the filled prescription order in the memory 158. Receiving the selection of the filled prescription order may include receiving the identification information of the filled prescription order or the identification information of the storage bag 70 including the filled prescription order. In one example, receiving the selection of the filled prescription order includes scanning, using the stock scanner 102, a barcode on the filled prescription order or the storage bag 70 including the filled prescription order. Once the filled prescription order is identified, the pharmacist may place the storage bag 70 on the stock holder 178. In examples where a stock transport loop is used instead of the stocking station 174, the pharmacist may place the storage bag 70 on a hook or transport block of the stock transport assembly.

The method 600 also includes determining, using the electronic processor 154, a transport block 54 corresponding to the filled prescription order (at block 620). In some examples, the electronic processor 154 automatically finds an empty transport block 54 to stock the filled prescription order. The method 600 includes transferring, using the stocking mechanism 182, the filled prescription order to the transport assembly 38 (at block 630). The electronic processor 154 controls the stocking mechanism 182 to transfer the storage bag 70 including the filled prescription order from the stock holder 178 (or the stock transport loop) to a transport block 54 of the transport assembly 38.

The method 600 includes relating, using the electronic processor 154, the filled prescription order with the corresponding transport block 54 (at block 640). The electronic processor 154 may store a mapping in the memory 158 corresponding identification information of the filled prescription orders (or the storage bags 70 including the filled prescription orders) with the identification information of the transport blocks 54 where the filled prescription orders are stored. This mapping is available for later reference when dispensing the filled prescription order or when returning the filled prescription order. The method 600 may be repeated for every filled prescription order being stocked in the order delivery and storage system 34.

The examples of FIGS. 16-18 allow the order delivery and storage system 34 to be stocked without having to stop the transport assembly 38. Specifically, the electronic processor 154 monitors the movement of the transport assembly 38 and actuates the stocking mechanism 182 when the transport assembly 38 is idle to transfer the storage bags 70 from a holding location (e.g., the stock holder 178 or the stock transport loop) to the transport assembly 38.

The above-described order delivery and storage system 34 may be retrofit into existing pharmacies or built as part of a new pharmacy. The order delivery and storage system 34 may also be scaled, depending on the size of the pharmacy. For example, the transport assembly 38 and the enclosure 42 may be configured to store between 50 and 10,000 prescription orders. In other configurations, the transport assembly 38 and the enclosure 42 may be configured to store even more prescription orders. In addition, the order delivery and storage system 34 may include any number of dispensing tubes 50 and kiosks 30.

Various features and advantages of the disclosure are set forth in the following claims.

## Claims

1. A pharmacy order delivery and transport system comprising:
an enclosure;
a transport assembly provided in the enclosure and forming a continuous loop, the transport assembly movable through the enclosure and configured to transport a plurality of filled prescription orders;
a transport actuator configured to drive the transport assembly;
a dispensing opening provided on the enclosure; and
an electronic processor system coupled to the transport actuator and configured to
receive a selection of a filled prescription order from the plurality of filled prescription orders for dispensing,
determine a location of the filled prescription order on the transport assembly;
operate, using the transport actuator, the transport assembly to align the location of the filled prescription order with the dispensing opening, and
dispense, via the dispensing opening, the filled prescription order.

2. The pharmacy order delivery and transport system of claim 1, wherein the transport assembly includes a plurality of transport blocks connected together to form the continuous loop.

3. The pharmacy order delivery and transport system of claim 2, wherein the transport assembly includes a track, wherein each transport block of the plurality of transport blocks includes a transport hook and a wheel, and wherein the wheel is configured to move along the track.

4. The pharmacy order delivery and transport system of claim 2 or claim 3, wherein the location of the filled prescription order corresponds to identification information of a transport block of the plurality of transport blocks used to store the filled prescription order.

5. The pharmacy order delivery and transport system of any of claims 2 to 4, wherein the filled prescription order is a first filled prescription order, and wherein the electronic processor system is further configured to:
receive a selection of a second filled prescription order;
determine a transport block of the plurality of transport blocks corresponding to the second filled prescription order; and
relate the second filled prescription order with the transport block, wherein a location of the second filled prescription order corresponds to identification information of the transport block.

6. The pharmacy order delivery and transport system of any of claims 2 to 5, further comprising:
a scanner coupled to the enclosure and configured to identify a location of the transport assembly,
wherein the location of the transport assembly corresponds to identification information of a transport block in front of the scanner, and
wherein locations of the plurality of transport blocks are determined based on the location of the transport assembly.

7. The pharmacy order delivery and transport system of any preceding claim, wherein operating the transport assembly to align the location of the filled prescription order with the dispensing opening includes operating the transport assembly such that the filled prescription order is directly above the dispensing opening.

8. The pharmacy order delivery and transport system of any preceding claim, wherein the dispensing opening is a first dispensing opening and the filled prescription order is a first filled prescription order, further comprising:
a second dispensing opening in communication with a return container,
wherein the electronic processor system is configured to:
determine that a second filled prescription order of the plurality of filled prescription orders is past a pick-up by date;
determine a location of the second filled prescription order on the transport assembly;
operate the transport assembly to align the location of the second filled prescription order with the second dispensing opening; and
return, via the second dispensing opening, the second filled prescription order to the return container.

9. The pharmacy order delivery and transport system of any preceding claim, wherein:
(i) the enclosure is configured to be coupled to a ceiling of a building; and/or
(ii) the enclosure is made of clear or translucent materials.

10. The pharmacy order delivery and transport system of any preceding claim, wherein the enclosure is made of modular sections, and optionally wherein the modular sections include straight sections and bent sections.

11. The pharmacy order delivery and transport system of any preceding claim, wherein the enclosure includes a plurality of spaced apart doors to selectively provide access to an interior of the enclosure.

12. A pharmacy order delivery and transport system comprising:
an enclosure; and
a transport assembly provided in the enclosure and configured to transport a plurality of filled prescription orders, the transport assembly including
a track extending continuously throughout the enclosure,
a plurality of transport blocks connected together to form a continuous loop, each transport block of the plurality of transport blocks including a transport hook and a wheel configured to move along the track, the transport hook configured to receive a storage bag containing a filled prescription order of the plurality of filled prescription orders, and
a transport actuator configured to drive the plurality of transport blocks along the track.

13. The pharmacy order delivery and transport system of claim 12, further comprising:
(i) an electronic processor system coupled to the transport actuator and configured to:
receive a desired location of the transport assembly;
determine a current location of the transport assembly; and
control the transport actuator to move the transport assembly to the desired location based on the current location of the transport assembly, and optionally
(ii) further comprising:
a scanner coupled to the enclosure and configured to identify a location of the transport assembly,
wherein the location of the transport assembly corresponds to identification information of a transport block in front of the scanner,
wherein locations of the plurality of transport blocks are determined based on the location of the transport assembly, and
wherein operating the transport assembly to the location of the filled prescription order includes operating the transport assembly such that the filled prescription order is directly above a dispensing opening of the pharmacy order delivery and transport system as determined based on the location of the transport assembly.

14. The pharmacy order delivery and transport system of claim 13, wherein:
(a) the filled prescription order is a first filled prescription order, and wherein the electronic processor system is further configured to
receive a selection of a second filled prescription order;
determine a transport block of the plurality of transport blocks corresponding to the second filled prescription order; and
relate the second filled prescription order with the transport block, wherein a location of the second filled prescription order corresponds to identification information of the transport block; or
(b) the filled prescription order is a first filled prescription order, further comprising:
a dispensing opening provided on the enclosure and in communication with a return container, and
wherein the electronic processor system is further configured to
determine that a second filled prescription order of the plurality of filled prescription orders is past a pick-up by date,
determine a location of the second filled prescription order on the transport assembly,
operate the transport assembly to align the location of the second filled prescription order with the dispensing opening, and
return, via the dispensing opening, the second filled prescription order to the return container.

15. The pharmacy order delivery and transport system of any of claims 13 or 14, wherein the filled prescription order is a first filled prescription order, further comprising:
a dispensing opening provided on the enclosure, and
wherein the electronic processor system is further configured to
receive a selection of a second filled prescription order from the plurality of filled prescription orders for dispensing,
determine a location of the second filled prescription order on the transport assembly,
operate, using the transport actuator, the transport assembly to align the location of the second filled prescription order with the dispensing opening, and
dispense, via the dispensing opening, the filled prescription order, and optionally
wherein the location of the second filled prescription order corresponds to identification information of a transport block of the plurality of transport blocks used to store the second filled prescription order.
